# EUROPEAN PATENT APPLICATION

(11) **EP 4 553 060 A1**
(43) Date of publication of application: **14.05.2025**
(21) Application number: 23208844.3
(22) Date of filing: 09.11.2023
(51) Int. Cl.: C07C 1/20, C07C 1/24, C07C 29/151, C07C 45/34, C07C 51/235, C07C 67/05

(54) **VINYL ACETATE HAVING A NATURAL ABUNDANCE OF CARBON-14 FROM NON-FOSSIL RESOURCES**

(71) Applicant: BASF SE, 67056 Ludwigshafen am Rhein (DE)
(72) Inventor: WEISS, Thomas, 67056 Ludwigshafen am Rhein (DE); WITTELER, Helmut, 67056 Ludwigshafen am Rhein (DE); HUEFFER, Stephan, 67056 Ludwigshafen am Rhein (DE); TROPSCH, Juergen, 67056 Ludwigshafen am Rhein (DE); KOENIG, Christian, 67056 Ludwigshafen am Rhein (DE); KRUEGER, Marco, 67056 Ludwigshafen am Rhein (DE); WIDMAIER, Ralf, 67056 Ludwigshafen am Rhein (DE)
(74) Representative: Schuck, Alexander

(57) **Abstract**

Process for making vinyl acetate from biomass, comprising the step of reacting (I) ethylene with (II) acetic acid to give vinyl acetate, wherein
(I) ethylene is provided, starting from biomass, by a process comprising
(a) producing carbon oxides from biomass, or capturing carbon dioxide from ambient air, optionally followed by electrochemical reduction of carbon dioxide to carbon monoxide;
(b) electrochemical reduction of carbon oxides from step (a) to give ethylene; and/or
(c1) reacting hydrogen and carbon oxides from step (a) in the presence of a catalyst to give methanol,
(c2) reacting methanol from step (c1) to form ethylene; and/or
(d1) producing ethanol from biomass by fermentation,
(d2) dehydrogenating ethanol from step (d1) to give ethylene; and/or
(e) directly producing ethylene from biomass by fermentation;

(II) acetic acid is provided, starting from biomass or carbon dioxide captured from ambient air, by a process comprising
(f) reacting methanol from step (c1) with carbon monoxide to give acetic acid; and/or
(g1) reacting part of the ethylene from steps (b), (c2), (d2) or (e) with oxygen and water to give acetaldehyde, and
(g2) reacting acetaldehyde from step (g1) with oxygen to give acetic acid; and/or
(h) oxidative fermentation of ethanol from step (d1) to give acetic acid; and/or
(i) producing acetic acid from biomass by biomass pyrolysis.

## Description

The present invention relates to a vinyl acetate having a natural abundance of carbon-14 from non-fossil resources, preferably from biomass, the vinyl acetate obtained thereby as well as to its use.

In the chemical industry, methanol serves as a raw material in the production of olefines, formaldehyde, acetaldehyde, acetic acid, methyl acetate, acetic anhydride and vinyl acetate. The conventional production method involves a catalytic process using fossil feedstock such as natural gas or coal.

Synthesis gas (syngas) for the production of methanol can be produced from many sources, including natural gas, coal, biomass, or virtually any hydrocarbon feedstock, by reaction with steam (steam reforming), carbon dioxide (dry reforming) or oxygen (partial oxidation).

Syngas is produced from solid feedstocks via coal gasification. Coal is reacted thereby in a mixture of partial oxidation with air or pure oxygen and gasification with water vapor to give a mixture of carbon monoxide and hydrogen.

The most important gaseous educt for producing syngas is natural gas, which is reacted with water vapor via steam reforming. Natural gas provides the highest hydrogen to carbon monoxide ratio, according to the equation

CH₄ + H₂O → CO + 3 H₂,

Also liquid educts, such as light naphtha cuts, can be reacted, after sulfur removal, with water vapor via steam reforming.

To produce methanol, the ratio of carbon monoxide to hydrogen in the synthesis gas is adjusted to meet the reaction equation

CO + 2 H₂ → CH₃OH,

While natural gas is used for the methanol production in North America and in Europe, syngas production is based mainly on coal in China and South Africa.

Most of the vinyl acetate is produced via the vapor-phase reaction of ethylene and acetic acid over a noble-metal catalyst, usually palladium.

Acetic acid can be produced by carbonylation of methanol by the Monsanto-process or the Cativa-process. Acetic acid can be alternatively produced by reacting ethylene with oxygen and water to give acetaldehyde, and further reaction of acetaldehyde with oxygen to give acetic acid.

Ethylene can be obtained by a methanol to olefin-process (MTO-process) from methanol.

Polyvinyl acetate (abbreviations PVAC, PVA) is a thermoplastic. Polyvinyl acetate is an amorphous, odourless and tasteless plastic with high light and weather resistance. It is combustible, but not easily flammable. The glass transition temperature of the homopolymer varies between 18 and 45 °C depending on the degree of polymerization. The electrical, mechanical and thermal properties are also largely dependent on the degree of polymerization. The minimum film forming temperature of homopolymer dispersions is about 15 to 18 °C. Polyvinyl acetate is processed in the form of solutions in organic solvents or as a dispersion.

PVA is used as a binder in paints and varnishes. The plastic is also used as an adhesive, for example as white glue (wood glue), wallpaper paste or parquet adhesive. The universal adhesive UHU, which is well known in Germany, is a forty percent solution of polyvinyl acetate in methyl acetate and acetone. Simple craft glue also often contains mainly polyvinyl acetate and is then called vinyl glue. Other applications include paper manufacturing and coating, textile impregnation, carpet backing or modification of plaster and concrete. In addition, PVA is often a component of chewing gum masses and is used for coating cheese or sausage.

Vinyl acetate is able to form copolymers with a variety of monomers such as ethylene, maleic anhydride, maleic esters, vinyl ethers and allyl ethers. From that copolymers especially the ethylene vinyl acetate copolymers (EVAC) are prepared in huge amounts to produce thermoplastic elastormers and thermoplastic materials. Depending on the ethylene : vinyl acetate ratio, a broad variety of applications can be addressed. Vinyl acetate contents of up to 7 % are almost exclusively used to improve the properties (especially to increase the elongation at break) of films. About half of EVAC production is made with a vinyl acetate content of less than 7 wt.%. EVAC with a vinyl acetate content of 7 to wt.18 % is also often used as an exclusive material for special applications. Examples include cold-resistant pull-out spouts for canisters, films for agriculture and horticulture, shrink-wrap films (office supplies, solar panels), shower curtains, floor coverings, roofing membranes and electrical cables. The class of EVAC above with up to 28 wt.% vinyl acetate is mainly used as a hot melt adhesive, which in turn is used for fibre bonding of very high quality tufted carpets and needle punched nonwovens, for perfect binding in book production and for manual use with hot melt glue guns. With vinyl acetate contents of more than 30 wt% up to 90 wt.%, a rubber-like thermoplastic elastomer is produced. It is mainly used for shoe soles or as a polymer blend together with other elastomers.

Furthermore the vinyl acetate monomer (VAM) can be used to prepare watersoluble graft-polymers on polyether substrates. These graft-copolymers are used in laundry detergent compositions for antigraying, e.g. Sokalan HP22 (DE3711298; BASF SE).

Vinyl acetate is produced form precursor base stocks such as ethylene and acetic acid. These base stocks typically derive from fossil carbon base stocks, such as oil or natural gas. Since the carbon source leads to an unwanted CO₂ balance there is need for production of vinyl acetate monomer (VAM) and polyvinyl acetate and copolymers in a way that does not use fossil carbon sources. Vinyl acetate can form polymers such a polyvinyl acetate which is able to be hydrolyzed to biodegradable polyvinyl alcohol and biodegradable acetic acid. The production of vinyl acetate monomer VAM from non-fossil, renewable carbon sources has not been described before.

Stephen M. Mudge, Juergen Tropsch, Thierry Beaudouin, Christophe Séné, and Horacio Hormazabal, J Surfact Deterg (2020) 23: 771-780, report on the determination of the bio-based carbon content of surfactants, following a call of the European Committee for Standardization (CEN) for the development of a European standard (EN 17035) to define bio-based surfactants and enable quantification of the bio-based carbon content of surfactants based on radiocarbon analyses. The analytical approach described in the reference was tested through directly contracted analyses and through a round robin procedure at commercial facilities in Europe.

According to the reference, there are two major analytical approaches that might be used to determine the quantity of bio-based carbon present within any sample:
- Stable ¹³C isotope. The carbon isotope present within the surfactant molecule will reflect the initial carbon source and any transformations they may have undergone since formation. The δ¹³C for many crude oils is in the range of -23 to -28‰. This may be compared to -26 to -36‰ for terrestrial plant matter and -20 to -26‰ for unicellular algae. Since these ranges overlap, this may not be definitive in separating out the two sources.
- Radiocarbon (¹⁴C). Along with the ¹³C stable isotope, a very small proportion of the carbon in a molecule will be of the naturally occurring radioactive form, ¹⁴C, also called radiocarbon. The 14C atoms are formed in the upper atmosphere due to interactions between cosmic rays and nitrogen atoms. The natural abundance of ¹⁴C in compounds is around 1 part per trillion (ppt; 10¹²). This radioactive carbon isotope decays with a half-life of 5730 years such that after six half-lives, it is functionally undetectable in a sample. Carbon compounds that are derived from fossil sources such as oil or gas will contain no radiocarbon, as it will have decayed away during the millions of years needed to make such reserves. This contrasts with recently grown plant-based materials that do contain measurable amounts of ¹⁴C. Radioactive carbon can be measured using gas proportional counting, liquid scintillation counting, and accelerator mass spectrometry (AMS). The latter approach is the most sensitive of the three.

It is an object of the present invention to provide an environmentally friendly process for producing vinyl acetate. It is a further object of the invention to provide vinyl acetate that can be used for determining the content of bio-based vinyl acetate or bio-based vinyl alcohol derived therefrom by hydrolysis in vinyl acetate or vinyl alcohol containing polymers and copolymers. It is a still further object of the present invention to provide vinyl acetate for determining the origin of decay products released during decomposition of vinyl acetate or vinyl alcohol containing polymers or copolymers.

The object is solved by a process for making vinyl acetate from biomass, comprising the step of reacting (I) ethylene with (II) acetic acid to give vinyl acetate, wherein
(I) ethylene is provided, starting from biomass or ambient air, by a process comprising
   (a) producing carbon oxides from biomass, or capturing carbon dioxide from ambient air, optionally followed by electrochemical reduction of carbon dioxide to carbon monoxide;
   (b) electrochemical reduction of carbon oxides from step (a) to give ethylene; and/or
   (c1) reacting hydrogen and carbon oxides from step (a) in the presence of a catalyst to give methanol,
   (c2) reacting methanol from step (c1) to form ethylene; and/or
   (d1) producing ethanol from biomass by fermentation, and
   (d2) dehydrogenating ethanol from step (d1) to give ethylene; and/or
   (e) directly producing ethylene from biomass by fermentation;
(II) acetic acid is provided, starting from biomass or carbon dioxide captured from ambient air, by a process comprising
   (f) reacting methanol from step (c1) with carbon monoxide to give acetic acid; and/or
   (g1) reacting part of the ethylene from steps (b), (c2), (d2) or (e) with oxygen and water to give acetaldehyde, and
   (g2) reacting acetaldehyde from step (g1) with oxygen to give acetic acid; and/or
   (h) oxidative fermentation of ethanol from step (d1) to give acetic acid; and/or
   (i) producing acetic acid from biomass by biomass pyrolysis.

According to the invention, ethylene can be produced by one or more of steps (b), (c1)/(c2), (d1)/(d2) and (e). Acetic acid can be produced by one or more of steps (f), (g1)/(g2), (h) and (i).

In certain embodiments, ethylene is produced from one or more of steps (b), (c1)/(c2), (d1)/(d2) and (e), and acetic acid is produced form step (f). In one particular embodiment, the process comprises steps (c1), (c2) and (f). In another particular embodiment, the process comprises steps (c1), (c2), (g1) and (g2).

In certain other embodiments, ethylene is produced from one or more of steps (b), (c1)/(c2), (d1)/(d2) and (e), and acetic acid is produced form steps (g1)/((g2).

In certain other embodiments, ethylene is produced from one or more of steps (b), (c1)/(c2), (d1)/(d2) and (e), and acetic acid is produced form step (h).

In certain other embodiments, ethylene is produced from one or more of steps (b), (c1)/(c2), (d1)/(d2) and (e), and acetic acid is produced form step (i).

There are three naturally occurring isotopes of carbon on Earth: carbon-12 (¹²C), which makes up 99% of all carbon on Earth; carbon-13 (¹³C), which makes up 1%; and carbon-14 (¹⁴C), which occurs in trace amounts, making up about 1 or 1.5 atoms per 10¹² atoms of carbon in the atmosphere. Carbon-12 and carbon-13 are both stable, while carbon-14 is unstable and has a half-life of 5700±30 years. Carbon-14 decays into nitrogen-14 (¹⁴N) through beta decay. The primary natural source of carbon-14 on Earth is cosmic ray action on nitrogen in the atmosphere, and it is therefore a cosmogenic nuclide.

The natural abundance of carbon-14 (14C) is approximately 1 ppt (parts per trillion; 10⁻¹²; 10⁻¹⁰ atom-%), in general 0.5 to 2.0 ppt, based on the total carbon content.

In step (a), carbon oxides are produced from biomass. A suitable biomass is lignocellulsoic biomass, for example lignocellulosic waste biomass.

CO₂ or CO can be converted with hydrogen to methanol for further processing to ethylene. Alternatively, CO₂ or CO can be converted to ethylene via electrified conversion processes, i.e., converted to C₂ products electrochemically. CO₂/CO electrolysis can be implemented via membrane electrode assembly technology. Biomass can be used as renewable carbon feedstock for electrochemical processes.

Ethylene production via biomass gasification and electrochemical CO reduction is described by Klüh et al. (2023), Assessment of electrified ethylene production via biomass gasification and electrochemical CO reduction, Front. Energy Res. 11:1129076. doi:
10.3389/fenrg.2023.1129076, with further references:
The electrochemical reduction of CO₂ (CO₂R) can produce a wide range of products like ethylene, ethanol, acetic acid, propanol, methanol, or formic acid. The utilization of a two-step process from CO₂ to C₂ products via CO as intermediate is considered beneficial compared to direct CO₂ conversion. Reduction of CO to C₂ products (COR) can be combined with biomass gasification or biomass combustion.

In certain embodiments, carbon oxides are produced from biomass in step (a) via gasification of the biomass. In certain other embodiments, carbon oxides are produced from biomass in step (a) via combustion of the biomass.

The combination of biomass-based and electrified processes, termed Power-/Biomass-to-X (PBtX), has been widely investigated in recent years. Two main routes, both based on the electrochemical reduction of CO, utilize lignocellulosic waste biomass as feedstock, but differentiate in terms of the employed thermochemical treatment: gasification or combustion. In case of the gasification route, the biomass is dried and gasified in an oxygen and steam blown fluidized bed gasifier. The CO rich stream is sent to the electrochemical CO reduction. From the purge stream of the membrane separation, hydrogen is recovered via pressure swing adsorption (PSA). The leftover gas is used for heating the reformer. In case of the combustion-based route, biomass is combusted in a fluidized bed combined heat and power (CHP) plant producing heat and electricity for the operation of the processes. CO₂ is separated from the flue gas with a monoethanolamine (MEA) wash. See Klüh et al. (2023), Front. Energy Res. 11:1129076.

The CO₂ is further converted to CO in a CO₂ electrolysis unit. The CO is then further processed in the electrochemical CO reduction unit.

During electrochemical reduction, the CO is converted in an electrochemical cell to ethylene, acetic acid, ethanol, oxygen, and hydrogen. Ethanol and acetic acid are separated from the electrolyte via rectification. Oxygen is easily separated from the liquid phase of the anode. Unreacted CO, H₂, and ethylene from the cathode are separated via PSA. The unconverted CO together with traces of H₂ and ethylene are recycled to the electrochemical cell.

Gasification of biomass can be carried out as described in by Klüh et al. (2023), *Front. Energy Res.* 11:1129076. The gasification route consists of biomass drying and gasification, followed by COR and product separation. Biomass is dried before it can be further processed in gasification. The biomass dryer can be for instance a belt dryer operating at a temperature level of 120°C. The water content is reduced e. g. from 35 to 15 wt.%. The dried biomass is gasified in an oxygen blown fluidized bed gasifier. The gasifier can comprise two reactors. In the decomposition reactor (RYield), the biomass is broken down into its elements, while the subsequent reactor finds the chemical equilibrium.

The combustion-based route consists of biomass combustion with CO₂ capture, conversion of CO₂ by electrolysis, and electrochemical conversion of CO followed by product separation. The CO₂ is separated from the flue gas of the biomass CHP plant. Post-combustion capture by absorption with MEA for CO₂ separation can be used as an established technology for capturing CO₂ from power plant flue gases.

In certain embodiments, carbon dioxide contained in the product gas obtained from biomass gasification or combustion is electrochemically reduced to carbon monoxide.

CO₂ can be converted electrochemically to CO according Equation (1). On the cathode, carbon dioxide is reduced to carbon monoxide, while oxygen is formed on the anode. CO is separated from the product stream and the unreacted CO₂, also containing some CO, can be recycled back to the electrolysis cell.

2CO₂ → 2CO +O₂ (1)

Electrochemical reduction of the carbon dioxide contained in the gas stream provided in step (a) is carried out to obtain a gas stream containing carbon monoxide, optionally carbon dioxide and optionally hydrogen.

A large number of methods for the electrochemical production of CO from CO₂ have been proposed. Most of these methods are at a very early stage of development. High-temperature electrolysis in solid oxide cells is a CO₂ electrolysis technology that is approaching commercialization and for which long-term durability exceeding a year on stream has been demonstrated.

A review of two alternative electrochemical technologies for CO production, low-temperature and molten carbonate electrolysis, is given in Reiner Küngas (2020), J. Electrochem. Soc. 167 044508, with further references:
Regardless of the choice of technology, an electrolysis cell always has at least three components: two electrodes in contact with an electrolyte. The electrolyte is either a liquid or a solid material that can conduct ions (e.g. protons, hydroxide ions, oxide ions, carbonate or bicarbonate ions), but that is impermeable to electrons. The ionic conductivity of the electrolyte depends strongly on temperature and the choice of the electrolyte material thereby determines the operating temperature of the cell. When an external voltage is applied between the two electrodes, electrochemical reactions start to occur. The electrode where the reduction of reactants (e.g. CO₂ to CO) takes place is called the cathode. The electrode where the oxidation of reactants (e.g. OH- to O₂ and H₂O or O²⁻ to O₂) occurs is referred to as the anode.

In solid oxide electrolysis cells (SOECs), the electrolyte is a solid ceramic material. At temperatures above around 600 °C, electrolyte materials start to conduct oxide ions, but remain impermeable to gaseous oxygen and to electrons. As the ionic conductivity of electrolyte materials increases exponentially with temperature, the operating temperature of SOECs is typically chosen to be between 700 °C and 900 °C. Commonly used materials include stabilized zirconias, such as yttria-stabilized zirconia (YSZ, a solid solution of Y₂O₃ and ZrO₂) and scandia-stabilized zirconia (ScSZ), as well as doped cerias, such as gadolinia-doped ceria (abbreviated either as GDC or CGO) or samaria-doped ceria (SDC or CSO).

CO₂ is fed to the cathode side of the cell via gas channels, which help to distribute the gas across the cell. In the porous cathode (also referred to as the fuel electrode), carbon dioxide is reduced to carbon monoxide, following the reaction

CO₂ + 2e- -> CO + O²⁻

The electrons for the reaction are provided by an external power supply. The oxide ions (O²⁻) formed in the reaction are incorporated into the electrolyte and traverse through the electrode into the anode (also called the oxygen electrode), where the ions are oxidized into molecular oxygen according to the reaction

O²⁻ -> ½ O₂ + 2 e-

The formed oxygen gas is led out of the cell via gas channels. It is important to note that as long as pure CO₂ (or a mixture of CO and CO₂) is fed to the fuel electrode, the formed product will be free of H₂ and H₂O.

Composites of metallic Ni and either CGO or YSZ are the most commonly used materials in SOEC fuel electrodes. Typical oxygen electrode materials for SOECs include doped perovskites of lanthanides and transition metals, such as Sr-doped LaMnOs (LSM), Sr-doped La(Fe,Co)O₃ (LSCF), Sr-doped SmCoOs (SSC) and many others.

In molten carbonate electrolysis cells (MCECs), the electrolyte is a carbonate melt. A combination of molten Li₂O/Li₂CO₃ electrolyte, a titanium cathode and a graphite anode has been shown to give promising results. In this material system, carbonate ions are reduced to CO and oxide ions at the cathode (CO₃²⁻ + 2 e⁻ -> CO + 2 O²⁻), while oxide ions are oxidized to gaseous oxygen at the anode (O²⁻ -> ½ O₂ + 2 e).

In effect, Li₂CO₃ is electrochemically converted into Li₂O on the cathode, thereby increasing the Li₂O/Li₂CO₃ ratio in the melt. As the oxide content in the electrolyte increases, new CO₂ can chemically incorporated into the mixture. The ratio of Li₂O/Li₂CO₃ in the electrolyte is thereby a function of both by the applied current density and the concentration of CO₂ above the melt. A key advantage of MCECs is that the CO₂ feed and the CO and O₂ products do not mix, allowing pure gases to be extracted from the cell. Additionally, the method is only mildly affected by SO₂-content in the feed gas, and can potentially use dilute and humid CO₂ streams, suggesting that industrial flue gases may be used as feed.

In low-temperature electrolysis cells, CO₂ reduction is carried out in aqueous solutions. The electrolytes can either be solid ion-selective membranes (e.g. Nafion, Sustainion), aqueous solutions (e.g. KHCO₃), or combinations thereof. Most of the low temperature electrolysis cells today operate in alkaline or pH-neutral conditions.

Most of the electrode development work on electrochemical CO₂ reduction has been carried out using a cell configuration where both electrolyzer electrodes are immersed in electrolyte solutions (the anolyte and catholyte, respectively). Such an electrode configuration is referred to as the H-cell. On the anode of such a cell, oxygen evolution proceeds according to either of the two reactions:

2 OH- -> ½ O₂ + H₂O + 2 e-

H₂O -> ½ O₂ + 2 H⁺ + 2 e-

On the cathode, CO₂ is electrochemically reduced to CO:

CO₂ + H₂O + 2 e- -> CO + 2 OH-

Commonly, CO production is accompanied by hydrogen evolution, which in alkaline media proceeds via reaction

2 H₂O + 2 e- -> 2 OH- + H₂

The delivery of gas-phase CO₂ to the cathode and the use of gas-diffusion electrodes present means of overcoming mass transport limitations in low-temperature electrolysis systems. In some designs, gas-diffusion electrodes are employed in both electrodes. IrO₂ is used almost exclusively as the catalyst material on the anode side of aqueous electrolysis cells. Cathode materials for the production of CO typically include Ag and Au, with catalyst supports shown to play an important role for activity, selectivity, and stability.

The electrical power necessary for the electrochemical reduction of CO₂ to CO is generated at least in part from non-fossil, renewable resources. In other words, part of the electrical power can still be produced from fossil fuels, preferably from natural gas, since combustion of natural gas causes much lower carbon dioxide emission per Megajoule of electrical energy produced than combustion of coal. However, the portion of electrical energy produced from fossil fuels should be as low as possible, preferably ≤ 50%, preferably ≤ 30%, most preferably ≤ 20%.

The electrical power from non-fossil resources used in carbon dioxide according to the invention can be generated by nuclear energy. Nuclear energy is considered renewable by the European Commission, as long as certain preconditions (i. a. safe long-term storage of nuclear waste) are fulfilled.

The electrical power from non-fossil resources used in carbon dioxide according to the invention is preferably generated from wind power, solar energy, biomass, hydropower and geothermal energy.

In some other preferred embodiments, the carbon dioxide that is provided in step (b) is captured from ambient air.

Direct air capture (DAC) is a process of capturing carbon dioxide (CO₂) directly from the ambient air and generating a concentrated stream of CO₂ for sequestration or utilization or production of carbon-neutral fuel. Carbon dioxide removal is achieved when ambient air makes contact with chemical media, typically an aqueous alkaline solvent or sorbents. These chemical media are subsequently stripped of CO₂ through the application of energy (namely heat), resulting in a CO₂ stream that can undergo dehydration and compression, while simultaneously regenerating the chemical media for reuse.

Dilute CO₂ can be efficiently separated using an anionic exchange polymer resin called Marathon MSA, which absorbs air CO₂ when dry, and releases it when exposed to moisture. A large part of the energy for the process is supplied by the latent heat of phase change of water. Other substances which can be used are metal-organic frameworks (or MOFs). Membrane separation of CO₂ rely on semi-permeable membranes.

Carbon dioxide captured from ambient air can likewise be electrochemically reduced by the methods described above before being used in step (b) or in step (c1).

In step (b), carbon monoxide is converted to C₂ products in an electrochemical reactor. Besides ethylene, ethanol, acetic acid, oxygen and hydrogen can be formed as products, according to equation (2) to (5):

2 CO + 2 H₂O → C₂H₄ + 2 O₂ (2)

2 CO + 3 H₂O → CH₃CH₂OH + 2 O₂ (3)

2 CO + 2 H₂O → CH₃COOH + O₂ (4)

2 H₂O → 2 H₂ + O₂ (5)

The process is described by Klüh et al. (2023), Front. Energy Res. 11:1129076, with further references.

In certain embodiments, a gas mixture containing carbon monoxide, hydrogen and carbon dioxide is reacted in step (c1) in the presence of a catalyst to give methanol.

The current world-scale technology for methanol synthesis is mostly based on the application of Cu/ZnO/AI2O3 (CZA) catalysts in either multi-tube reactors with boiling water as the cooling fluid, normally called isothermal reactors (e.g., the Lurgi process, the Linde process), or adiabatic reactors with intermediate cold syngas quenching, generally named quench reactors (e.g., ICI and the Casale process, the Haldor Topsoe process). Less common but also industrially applied are the adiabatic reactors with intermediate cooling (e.g., the Kellogg process, the Toyo process). Normally, temperatures between 200 and 300 _{°}C and pressures between 50 and 100 bar are applied. See Bozzano, G.; Manenti, F. Efficient methanol synthesis: Perspectives, technologies and optimization strategies. Prog. Energy Combust. Sci. 2016, 56, 71-105; and Ott, J.; Gronemann, V.; Pontzen, F.; Fiedler, E.; Grossmann, G.; Kersebohm, D.B.; Weiss, G.; Witte, C. Methanol. In Ullmann's Encyclopedia of Industrial Chemistry; Wiley: New York, NY, USA, 2012.

In certain other embodiments, hydrogen and carbon dioxide are reacted in step (c1) in the presence of a catalyst to form methanol.

An overview of suitable catalyst systems is given by https://link.springer.com/article/10.1007/s40974-020-00156-4" \I "auth-Kristian-Stangeland, Hailong Li & Zhixin Yu, Energy, Ecology and Environment volume 5, pages 272-285 (2020). Multi-component catalyst systems are required for this process. The interaction between components is essential for high activity and selectivity of CO₂-to-methanol catalysts. This has been demonstrated by numerous catalyst systems comprised of various metals (i.e., Cu, Pd, Ni) and metal oxides (i.e., ZnO, ZrO₂, In₂O₃). These complex systems can contain a mixture of metallic, alloy, and metal oxide phases. The most promising catalyst systems for large-scale industrial processes are currently Cu-based and In-based catalysts due to their superior catalytic performance.

A process for the CO₂-to-methanol synthesis can be carried out, for example, by the method known from DE-A-42 20 865, which produces methanol under the influence of silent electrical discharges.

Alternatively, methanol synthesis can also be carried out in a thermal reactor under pressure and elevated temperature and in the presence of a copper-based catalyst, as described in DE 43 32 789 A1 and DE 19739773 A1.

Typical catalysts are described, for example, in the publication by N.Kanoun et al. "Catalytic properties of Cu based catalysts containing Zr and/or V for methanol synthesis from a carbon dioxide and hydrogen mixture" in CATALYSIS LETTERS 15,(1992) 231-235. Potential catalysts like CuO/ZnO and Cu-ZnO-Al₂O₃ are also described by R. M. Navarro et al. "Methanol Synthesis from CO2: A Review of the Latest Developments in Heterogeneous Catalysis" Materials (2019), 12, 3902 and in "Catalytic carbon dioxide hydrogenation to methanol: A review of recent studies" in chemical engineering research and design 92 (2014) 2557-2567.

Recently a high selective catalysts In₂O₃/ZrO₂ was described for industrial relevant conditions. A typical range of industrially relevant conditions for the hydrogenation of CO₂ to methanol are T = 200 - 300°C, p = 10 - 50 MPa, and gas hourly space velocity (GHSV) of 16 000 - 48000 h⁻¹ (Angew. Chem. Int. Ed. 2016, 55, 6261 -6265).

The reaction of hydrogen and carbon dioxide in step (c1) can be carried out in the presence of a copper-zinc-alumina catalyst. If copper-zinc-alumina catalysts are employed, the preferred temperature is in the range of from 150 to 300°C, preferably 175 to 300°C and the preferred pressure is in the range of from 10 to 150 bar (abs).

In step (c2), methanol from step (c1) is reacted to yield ethylene.

C₂-C₄-olefins can be manufactures by a methanol to olefin-process (MTO-process) from methanol. A preferred process for the manufacture of C₂-C₄-olefins from methanol and optionally ethanol comprises the steps:
A) feeding a methanol and optionally ethanol comprising feed stream A in a dimethyl ether fixed bed reactor and catalytic conversion of methanol to give dimethyl ether, wherein a product stream A1 comprising dimethyl ether, methanol, water vapor and optionally ethanol and ethylene is obtained;
B) mixing of the stream A1 with at last one hydrocarbon recycle stream R comprising C₂-C₆-hydrocarbons and catalytic conversion in an olefin fixed bed reactor to yield a raw product stream B comprising C₂-C₄-olefines, C₅-C₆-hydrocarbons und C₇⁺-hydrocarbons;
C) cooling of the raw product stream B, wherein a hydrocarbon raw product stream C is obtained;
D) separating the hydrocarbon raw product stream C in a propylene containing value product stream, optionally an ethylene containing value product stream, a butene containing product stream, at least a C₅-C₆-hydrocarbon containing recycle stream and at least one C₆⁺-hydrocarbons containing side product stream;
E) recycling of a part of the C₂-C₄-olefins and at least a part of the C₅-C₆-hydrocarbons as one or more hydrocarbon recycle streams R in step B);
F) recovering a propylene containing value product stream, an ethylene containing value product stream and optionally a butene containing value product stream;
G) discharging the C₆⁺-hydrocarbons containing side product stream.

In an alternative process, ethanol is produced from biomass by fermentation in step (d1), and ethanol is dehydrogenated to give ethylene in step (d2).

Ethylene production by bioethanol dehydration, using forest or agro-industrial waste is a sustainable alternative to oil-based one. Gasification of lignocellulosic biomass is a thermochemical route to produce bioethanol. Using chemical catalysts, the synthesis gas generates ethanol together with a mixture of alcohols. Another option to produce ethanol is the fermentation of the synthesis gas.

Bioconversion of wood waste to bio-ethylene is described in Mendieta, C. M., Cardozo, R. E., Felissia, F. E., Clauser, N. M., Vallejos, M. E., and Area, M. C. (2021). "Bioconversion of wood waste to bio-ethylene: A review," BioResources, 16(2), 4411-4437, with further references:
The ethylene production process consists of pretreatment, enzymatic hydrolysis, fermentation, recovery by distillation, and dehydration. The conditions and type of pretreatment depend exclusively on the chemical composition of the feedstock, which has a significant influence on the enzymatic hydrolysis and following processes.

Before the production of bioethanol, it is critical to pretreat the lignocellulosic biomass. An efficient pretreatment should apply to various substrates using cheap and easily recoverable reagents, with low energy consumption, and low investment and maintenance costs, among other features. The reactions must favor the formation of sugars, avoid their loss by degradation, and limit the production of inhibitory products. The pretreatment process extracts the lignin and hemicellulose in order to increase the porosity of the material to improve cellulose accessibility to the enzymatic attack. The pretreatment process should also limit the degradation of other carbohydrates and, in the saccharification stage, avoid the formation of inhibitor products.

The cellulose that is obtained from the pretreatment can be transformed into ethanol in two steps. Initially, cellulose is depolymerized to glucose by hydrolysis, and then these sugars are fermented into ethanol. The common pretreatment strategies are separate hydrolysis and fermentation (SHF) and simultaneous hydrolysis and fermentation (SSF). In the SHF process, cellulose hydrolysis and glucose fermentation are accomplished separately, which allows each stage to occur at its optimum conditions. On the other hand, the SSF process requires only one reactor for hydrolysis and fermentation. The SSF process is the most feasible and cost-effective alternative to produce bioethanol considering the low generation of inhibitory products and the utilization of only one fermenter in the whole process, which reduces the investment costs.

In the petrochemical industry, dehydration of ethanol to ethylene is a usual process. Nevertheless, for the ethylene process, ethanol purity must be higher than 95 wt%. Unlike ethanol, bioethanol comes from a fermentation broth that contains microorganisms, nutrients, and reaction by-products. The obtained bioethanol must be purified by removing contaminants and additional water. For this, several methods combine distillation, adsorption, extraction, among others, such as separation by membrane pervaporation, extractive distillation, or heteroazeotropic distillation, which have been exhaustively described in the literature. For ethylene production, in particular, Very High Gravity (VHG) fermentation emerges as an interesting option to explore and optimize when using SHF, since it can improve fermentation performance and purify ethanol simultaneously. Considering that this is a stage of great energy consumption, it is one of the steps that require optimization to contribute to the economic viability of bio-ethylene.

Ethylene is formed by the highly endothermic intramolecular dehydration of ethanol, which eliminates one oxygen atom and two hydrogen atoms. The formation of ethylene occurs at temperatures between 350 °C and 500 °C. At low temperatures, intermolecular dehydration also produces diethyl ether, which can be sequentially dehydrated to form ethylene. For the dehydration reaction, catalysts are necessary. Alumina or alumina in conjunction with metal oxides as promoters, silica, clay, several metal oxides, phosphorus oxides, phosphates, molybdates, sulfuric acid, and zeolites, among others, have been studied as catalysts. Industrially, phosphoric acid and alumina have been used on a small-scale and they have been used to increase the ethylene selectivity. Alumina is the most commonly used catalyst for the dehydration of bioethanol, as it can withstand temperatures above 450 °C, but it deactivates quickly at temperatures below 300 °C. Zeolites are used to carry out the reaction because they do not require high temperatures, but at low-temperatures coke formation can produce the catalyst deactivation.

The effluents contain a high amount of water from the dehydration reaction, as well as ethanol feedstock and heat-carrying fluid. Water can be separated in a quench tower, and the residual ethanol and water-soluble oxygenates can be re-heated and distilled. The residual ethanol and diethyl ether (low water solubility) can be recovered and recycled to the feedstock, while the C₂H₄O can be burned in the furnace. The gas from the top of the quench tower primarily contains ethylene (90% to 99.5%), hydrocarbons, H₂, CO, CO₂, and oxygenates. The ethylene can be washed with cool water in a second tower removes the oxygenates, and caustic washing can remove CO₂ and acids.

In an alternative step (e), ethylene is directly produced from biomass by fermentation. Suitable fermentation processes are described in I. Pirkov, E. Albers, J. Norbeck, C. Larsson, Ethylene production by metabolic engineering of the yeast Saccharomyces cerevisiae, Metabolic Engineering, Volume 10, Issue 5, 2008, Pages 276-280; and Johansson, N., Quehl, P., Norbeck, J. et al., Identification of factors for improved ethylene production via the ethylene forming enzyme in chemostat cultures of Saccharomyces cerevisiae. Microb Cell Fact 12, 89 (2013).

Acetic acid can be produced by carbonylation of methanol in step (f). The process involves iodomethane as an intermediate, and occurs in three steps. A catalyst, metal carbonyl, is needed for the carbonylation (step 2).
1.

   CH₃OH + HI → CH₃I + H₂O
2.

   CH₃I + CO → CH₃COI
3.

   CH₃COI + H₂O → CH₃COOH + HI

Two related processes exist for the carbonylation of methanol: the rhodium-catalyzed Monsanto process, and the iridium-catalyzed Cativa process.

The Monsanto process operates at a pressure of 30-60 atm and a temperature of 150-200°C and gives a selectivity greater than 99%. The catalytically active species is the anion *cis-*[Rh(CO)₂I₂]⁻. The first organometallic step is the oxidative addition of methyl iodide to *cis-*[Rh(CO)₂I₂]⁻ to form the hexacoordinate species [(CH₃)Rh(CO)₂I₃]⁻. This anion rapidly transforms, via the migration of a methyl group to an adjacent carbonyl ligand, affording the pentacoordinate acetyl complex [(CH₃CO)Rh(CO)I₃]⁻. This five-coordinate complex then reacts with carbon monoxide to form the six-coordinate dicarbonyl complex, which undergoes reductive elimination to release acetyl iodide (CH₃C(O)I). The catalytic cycle involves two non-organometallic steps: conversion of methanol to methyl iodide and the hydrolysis of the acetyl iodide to acetic acid and hydrogen iodide.

The Cativa process is a further method for the production of acetic acid by the carbonylation of methanol. The technology is similar to the Monsanto process. The process is based on an iridium-containing catalyst, such as the complex [Ir(CO)₂I₂]⁻. The catalytic cycle for the Cativa process begins with the reaction of methyl iodide with the square planar active catalyst species to form the octahedral iridium(III) species [Ir(CO)₂(CH₃)I₃]⁻. This oxidative addition reaction involves the formal insertion of the iridium(I) centre into the carbon-iodine bond of methyl iodide. After ligand exchange of iodide for carbon monoxide, the migratory insertion of carbon monoxide into the iridium-carbon bond results in the formation of a species with a bound acetyl ligand. The active catalyst species is regenerated by the reductive elimination of acetyl iodide. The acetyl iodide is hydrolysed to produce the acetic acid product, in the process generating hydroiodic acid which is in turn used to convert the starting material methanol to the methyl iodide used in the first step.

Acetic acid can alternatively be produced by
(g1) reacting part of the ethylene from steps (b), (c1)/(c2), (d2) or (e) with oxygen and water to give acetaldehyde;
(g2) reacting acetaldehyde from step (g1) with oxygen to give acetic acid.

The Wacker process or the Wacker-Hoechst process refers to the oxidation of ethylene to acetaldehyde in the presence of palladium(II) chloride as the catalyst.

The net reaction can be described as follows:

[PdCl₄]^{2 -} + C₂H₄ + H₂O → CH₃CHO + Pd + 2 HCl + 2 Cl⁻

This conversion is followed by reactions that regenerate the Pd(ll) catalyst:

Pd + 2 CuCl₂ + 2 Cl ⁻ → [PdCl₄]²⁻ + 2 CuCI

2 CuCI + 1/2 O₂ + 2 HCl → 2 CuCl₂ + H₂O

Two routes are commercialized for the production of acetaldehyde: one-stage processes and two-stage processes.

In the one-stage process, ethene and oxygen are passed co-currently in a reaction tower at about 130°C and 400 kPa. The catalyst is an aqueous solution of PdCl₂ and CuCl₂. The acetaldehyde is purified by extractive distillation followed by fractional distillation. Extractive distillation with water removes the lights ends having lower boiling points than acetaldehyde (chloromethane, chloroethane, and carbon dioxide) at the top, while water and higher-boiling byproducts, such as acetic acid, crotonaldehyde or chlorinated acetaldehydes, are withdrawn together with acetaldehyde at the bottom.

In the two-stage process, reaction and oxidation are carried out separately in tubular reactors. Unlike the one-stage process, air can be used instead of oxygen. Ethylene is passed through the reactor along with catalyst at 105 - 110°C and 900 - 1000 kPa. Catalyst solution containing acetaldehyde is separated by flash distillation. The catalyst is oxidized in the oxidation reactor at 1000 kPa using air as oxidizing medium. Oxidized catalyst solution is separated and sent back to the reactor. Oxygen from air is used up completely and the exhaust air is circulated as inert gas. Acetaldehyde water vapor mixture is preconcentrated to 60 - 90% acetaldehyde by utilizing the heat of reaction and the discharged water is returned to the flash tower to maintain the catalyst concentration. A two-stage distillation of the crude acetaldehyde follows. In the first stage, low-boiling substances, such as chloromethane, chloroethane and carbon dioxide, are separated. In the second stage, water and higher-boiling by-products, such as chlorinated acetaldehydes and acetic acid, are removed and acetaldehyde is obtained in pure form overhead.

In both one- and two-stage processes the acetaldehyde yield is about 95%.

For further details, reference can be made to Marc Eckert, Gerald Fleischmann, Reinhard Jira, Hermann M. Bolt, Klaus Golka, Acetaldehyd, in Ullmann's Encyclopedia of Industrial Chemistry-7th ed, Vol.1, Kap. 4.3, p. 197.

Acetaldehyde can be oxidized with pure oxygen or with air in the presence of a redox catalyst to give acetic acid in step (e2). The oxidation can be carried out in the presence of cobalt or manganese acetate in acetic acid as solvent at temperatures of 50 - 70°C in bubble columns (Hoechst process).

Acetic acid can also be produced in step (h) by oxidative fermentation of ethanol from step (d1).

In a further alternative step (i), acetic acid can be produced from biomass by biomass pyrolysis.

Suitable processes for the production of acetic acid by biomass pyrolysis is described for instance in US2012/0172622 A1. Methods for producing acetic acid include pyrolyzing biomass, in general a lignocellulosic material such as wood, corn stover, and/or switch grass, to provide a pyrolysis reactor effluent. The biomass subjected to pyrolysis in an oxygen depleted environment, for example using Rapid Thermal Processing (RTP), can be any plant material, or mixture of plant materials. The methods also comprise separating at least a portion of the pyrolysis reactor effluent in a first separation stage (e.g., a quenching tower that includes quench liquid recycle) to provide first stage overhead and first stage bottoms products. The methods further comprise recovering the acetic acid from the first stage overhead product or the first stage bottoms product. Recovery can involve various processing steps, some or all of which may enrich a recovered intermediate or end product (e.g., a purified acetic acid product) in acetic acid and deplete the recovered product in other compounds (e.g., water and other oxygenates) produced from pyrolysis.

According to the invention, vinyl acetate is made by reacting (I) ethylene with (II) acetic acid, both being produced starting form biomass or from CO₂ captured from the atmosphere.

Most of the vinyl acetate is produced via the vapor-phase reaction of ethylene and acetic acid over a noble-metal catalyst, usually palladium. The reaction is typically carried out at 150 - 250°C, preferably 175 - 200°C and 5-9 bar pressure. The reaction is usually performed in the gas phase in a fixed bed tubular reactor using a supported catalyst. The amount of oxygen in the combined feed is within the range of 5 to 15 mol %. Preferably, the amount of acetic acid in the combined feed is within the range of 10 to 25 mol %. Preferably, the amount of ethylene in the combined feed is within the range of 65 to 80 mol %. Suitable catalysts include those known to the vinyl acetate industry. Preferably, the catalyst is a palladium-gold catalyst. Methods for preparing palladium-gold catalysts are known. For instance, U.S. Pat. No. 6,022,823 teaches how to prepare a palladium-gold catalyst which has high activity and selectivity. Preferably, the palladium-gold catalyst is supported on an inorganic oxide, such as alumina, silica, titania, and the like, and mixtures thereof.

The invention further concerns vinyl acetate with a natural abundance of carbon-14, obtainable by the processes as described herein.

The vinyl acetate according can have a ¹³C-content corresponding to a δ¹³C value of from -10 to -2.5 ‰, if the carbon dioxide of step (b) is captured from ambient air.

Vinyl acetate can be polymerized to polyvinyl acetate. Polyvinyl acetate is finally hydrolyzed to polyvinyl alcohol, which is biodegradable. Biodegradation of polyvinyl alcohol yields H₂O and CO₂, thereby closing the loop for a CO₂-neutral life cycle, if carbon oxides are prepared from biomass or CO₂ is taken from the atmosphere in step (a).

Poly(vinyl esters) are nontoxic but are degraded slowly in water, see Rinno, H. (2000), Poly(vinyl esters), in Ullmann's Encyclopedia of Industrial Chemistry, 7th ed, Vol. 28, Kap.8, p. 477 ff.

Polyvinyl alcohol is recognised as one of the very few vinyl polymers soluble in water that is susceptible to ultimate biodegradation in the presence of suitably acclimated microorganisms. Polyvinyl alcohol is nontoxic but is expected to biodegrade within 90 d under aquatic conditions, see Dominic Byrne et al., Biodegradability of polyvinyl alcohol based film used for liquid detergent capsules, Tenside Surf. Det. 58 (2021) 2; E. Chiellini et al., Prog. Polym. Sci. 28 (2003), pp. 963-1014.

For the copolymerization of bio-based vinyl acetate several monomer are of relevance, mostly preferred monomers are selected from other vinyl ester, e.g. vinyl propionate and vinyl laurate, vinyl pivalate, vinyl ester of branched alkyl carbonic acids. Also of importance and preferred are maleic acid esters, fumaric esters, allyl ethers, vinyl ethers such as vinyl ethylether, vinyl chloride, cyclic vinyl amides such as vinyl pyrrolidone. It is known that also ethylene and styrene can be copolymerized. Acrylic acid esters and methacrylic esters such as methacryl methylester can also be copolymerized efficiently if a third monomer of the monomers mentioned above is present.

It is also known that vinyl acetate is grafted efficiently on polyethers such as polyethylene glycol and other polyalkylene glycols, e.g., polyethylene oxide -block-polypropylene oxide or even randomized copolymers of alkylene oxides. In that mode block copolymers of polyvinyl acetate with the mentioned poly ethers are available. These block copolymers of polyvinyl acetate are useful as amphiphilic surface-active polymers in laundry and as emulsifier for formulations with agricultural actives.

Grafting of vinyl acetate occurs also efficiently on polysaccharides and other suitable polyhydroxy polymers e.g., polyvinyl alcohol, resulting in the formation of biodegradable blockcopolymers. Copolymers and terpolymers with combined vinyl acetate/vinyl alcohol monomer units are available through partial hydrolysis of the vinyl acetate copolymer.

The present invention also relates to polymers or copolymers of vinyl acetate, wherein the vinyl acetate has a natural abundance of carbon-14.

The present invention also relates to the use of vinyl acetate having a natural abundance of carbon-14 for determining the content of bio-based vinyl acetate or vinyl alcohol derived therefrom by hydrolysis in vinyl acetate or vinyl alcohol containing polymers and copolymers.

The present invention further relates to the use of vinyl acetate having a natural abundance of carbon-14 for determining the origin of decay products released during decomposition of vinyl acetate or vinyl alcohol containing polymers or copolymers.

Polymers and copolymers whose content of bio-based vinyl acetate or vinyl alcohol can be determined, or of which the origin of decay products released from the polymers and copolymers during decomposition can be determined include:
- polyvinyl acetate, polyvinyl alcohol, poly(vinyl acetate-co-vinyl alcohol);
- poly(vinyl acetate-co-vinyl alkyl ether), poly(vinyl acetate-co-allyl alkyl ether), poly(vinyl acetate-co-maleic acid derivatives), poly(vinyl acetate-co-acrylic acid derivatives), poly(vinyl acetate-co-methacrylic acid derivatives), poly(vinyl acetate-co-vinyl pyrrolidone) and their partially hydrolyzed derivatives (which are formally terpolymers);
- terpolymers of vinyl acetate, vinyl alcohol, and at least one further comonomer selected from vinyl laurate, vinyl versate, vinyl alkyl ethers, vinyl silyl ethers, vinyl pyrrolidone, vinyl caprolactone, maleic acid and maleic acid anhydrides and its derivative, e.g. methyl esters, Fumaric acid and fumaric acid derivatives, allyl alkyl ether, allyl alcohol esters, acrylic and methacrylic acid and derivatives thereof, e.g. amides and esters, keten derivatives such as cyclic ketene acetal monomers, and ethylene (the terpolymers can be obtained by the partial hydrolysis of the corresponding vinyl acetate copolymers);
- graft copolymers from vinyl acetate polymers, such as poly(vinyl acetate)-g-polyalkylene oxide, or poly(vinyl acetate)-g-polyglucane/polysaccharide derivative, and their derivatives obtained by hydrolysis or partial hydrolysis.

The carbon-14 carbon content in the (co)polymers or in the decay products can be determined using gas proportional counting, liquid scintillation counting, and accelerator mass spectrometry (AMS).

## Claims

1. Process for making vinyl acetate from biomass, comprising the step of reacting (I) ethylene with (II) acetic acid to give vinyl acetate, wherein
(I) ethylene is provided, starting from biomass or ambient air, by a process comprising
(a) producing carbon oxides from biomass, or capturing carbon dioxide from ambient air, optionally followed by electrochemical reduction of carbon dioxide to carbon monoxide;
(b) electrochemical reduction of carbon oxides from step (a) to give ethylene; and/or
(c1) reacting hydrogen and carbon oxides from step (a) in the presence of a catalyst to give methanol,
(c2) reacting methanol from step (c1) to form ethylene; and/or
(d1) producing ethanol from biomass by fermentation,
(d2) dehydrogenating ethanol from step (d1) to give ethylene; and/or
(e) directly producing ethylene from biomass by fermentation;
(II) acetic acid is provided, starting from biomass or carbon dioxide captured from ambient air, by a process comprising
(f) reacting methanol from step (c1) with carbon monoxide to give acetic acid; and/or
(g1) reacting part of the ethylene from steps (b), (c2), (d2) or (e) with oxygen and water to give acetaldehyde, and
(g2) reacting acetaldehyde from step (g1) with oxygen to give acetic acid; and/or
(h) oxidative fermentation of ethanol from step (d1) to give acetic acid; and/or
(i) producing acetic acid from biomass by biomass pyrolysis.

2. The process of claim 1, wherein carbon oxides are produced in step (a) from biomass by gasification or combustion of the biomass, preferably of lignocellulosic biomass.

3. The process of claim 1 or 2, wherein in step (a) carbon dioxide is electrochemically reduced to carbon monoxide by carbon dioxide electrolysis.

4. The process of any one of claims 1 to 3, wherein in step (c2) methanol is reacted to form ethylene in a methanol to olefin-process comprising the steps:
A) feeding a methanol and optionally ethanol comprising feed stream A in a dimethyl ether fixed bed reactor and catalytic conversion of methanol to give dimethyl ether, wherein a product stream A1 comprising dimethyl ether, methanol, water vapor and optionally ethanol and ethylene is obtained;
B) mixing of the stream A1 with at last one hydrocarbon recycle stream R comprising C₂-C₆-hydrocarbons and catalytic conversion in an olefin fixed bed reactor to yield a raw product stream B comprising C₂-C₄-olefines, C₅-C₆-hydrocarbons und C₇⁺-hydrocarbons;
C) cooling of the raw product stream B, wherein a hydrocarbon raw product stream C is obtained;
D) separating the hydrocarbon raw product stream C in a propylene containing value product stream, optionally an ethylene containing value product stream, a butene containing product stream, at least a C₅-C₆-hydrocarbon containing recycle stream and at least one C₆⁺-hydrocarbons containing side product stream;
E) recycling of a part of the C₂-C₄-olefins and at least a part of the C₅-C₆-hydrocarbons as one or more hydrocarbon recycle streams R in step B);
F) recovering a propylene containing value product stream, an ethylene containing value product stream and optionally a butene containing value product stream;
G) discharging the C₆⁺-hydrocarbons containing side product stream.

5. The process of any one of claims 1 to 4, wherein in step (d1) ethanol is produced by fermentation of lignocellulosic biomass.

6. The process of any one of claims 1 to 5, wherein ethylene is produced in step (b).

7. The process of any one of claims 1 to 6, wherein ethylene is produced in steps (c1) and (c2).

8. The process of any one of claims 1 to 7, wherein ethylene is produced in steps (d1) and (d2).

9. The process of any one of claims 1 to 8, wherein acetic acid is produced in step (f).

10. The process of any one of claims 1 to 9, wherein acetic acid is produced in steps (g1) and (g2).

11. The process of any one of claims 1 to 10, wherein acetic acid is produced in step (h).

12. The process of any one of claims 1 to 11, wherein acetic is produced in step (i).

13. Vinyl acetate having a natural abundance of carbon-14, obtainable by the process according to any one of claims 1 to 12.

14. Vinyl acetate of claim 13, having a carbon-14 content of 0.5 to 2.0 ppt, based on total carbon.

15. Polymers or copolymers of vinyl acetate, wherein the vinyl acetate has a natural abundance of carbon-14.

16. The use of vinyl acetate having a natural abundance of carbon-14 for determining the content of bio-based vinyl acetate or vinyl alcohol derived therefrom by hydrolysis in vinyl acetate or vinyl alcohol containing polymers or copolymers.

17. The of use of vinyl acetate having a natural abundance of carbon-14 for determining the origin of decay products released during decomposition of vinyl acetate or vinyl alcohol containing polymers or copolymers.
